(19)

**Europäisches Patentamt**
**European Patent Office**
**Office européen des brevets**

(11) **EP 3 640 946 A1**

(12) **EUROPEAN PATENT APPLICATION**

(43) Date of publication:
**22.04.2020 Bulletin 2020/17**

(51) Int Cl.:
**G16B 40/00** (2019.01)    G16C 20/70 (2019.01)

(21) Application number: **18200386.3**

(22) Date of filing: **15.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sartorius Stedim Data Analytics AB 903 33 Umeå (SE)**

(72) Inventors:
• **Sin Yee, Yau-Rose**
  **London,  NW9 5HG (GB)**

• **Vikström, Ernst Conny**
  **90651 Umea (SE)**
• **Rännar, Nils Erik Stefan**
  **90352 Umea (SE)**
• **Zehe, Christoph**
  **89584 Ehingen (Donau) (DE)**
• **Johansson, Erik Axel**
  **90421 Umea (SE)**
• **Mccready, Christopher Peter**
  **London, Ontario, Ontario N6C 1X7 (CA)**

(74) Representative: **Müller-Boré & Partner Patentanwälte PartG mbB Friedenheimer Brücke 21 80639 München (DE)**

(54) **MULTIVARIATE APPROACH FOR BIOLOGICAL CELL SELECTION**

(57)    According to an aspect, a computer-implemented method, a computer program and a process control device for selecting at least one set of target cells from multiple sets of candidate cells are provided. The method comprises receiving data collected from a plurality of processes, wherein each of the processes produces a distinct set of candidate cells. The method further comprises the received data including values of process parameters and process outputs of the processes, each of the process outputs being a product quality attribute or a key performance indicator for selecting the target cells. The method further comprises correlating the received data. The method further comprises receiving a selection of the process parameters and a selection of the process outputs. The method further comprises receiving multivariate evaluation criteria for the selected process parameters and/or the selected process outputs, the multivariate evaluation criteria including one or more of the following: weights for prioritization, prioritization ranges and/or targets. Each target is an extremum and/or a target value. The method further comprises calculating, via a multivariate selection function, scores for each one of the sets of candidate cells from the correlated data according to the multivariate evaluation criteria. The method further comprises ranking the sets of candidate cells according to the scores, and selecting at least one of the sets of candidate cells as the target cells using the ranking.

FIG 1

| Receive process parameter values and process outputs | S101 |
| Receive evaluation criteria, including at least one target | S103 |
| Determine distance between a value/output and the target | S105 |
| Magnify the distance via a continuous, non-linear function | S107 |
| Modify magnified distance based on priority | S109 |
| Aggregate modified distances corresponding to each paramater value and process output | S111 |

EP 3 640 946 A1

**Description**

The present application relates to cell, media, and process condition selection. More particularly, the application relates to/selecting at least one set of target cells from multiple sets of candidate cells. The cells may be clones.

The candidate cells may be from a pool of heterogeneous groups of cells. The cells may be transfected or transformed. It may be desirable to scale up from a first scale (microscale) to a second scale (macroscale) one or more orders of magnitude greater than the first scale, and for the target cells to be stable throughout a manufacturing life cycle, e.g., a drug manufacturing life cycle. The target cells may need to meet targets corresponding to predetermined product quality attributes or predetermined key performance indicators.

Often, large quantities of data may be generated for each set of candidate cells during the course of a process. The cells may be used as part of (or to host) a chemical, pharmaceutical, biopharmaceutical and/or biological product. In some cases, it may be desirable to identify problematic heterogeneities in the candidate cells or in parent cells.

Processes (e.g., carried out in a vessel or bioreactor) may generate huge amounts of data, particularly with respect to process parameters and/or conflicting (e.g., inversely correlated) product quality attributes. Problems regarding processing large amounts of data may be particularly acute in the case of a process control device capable of controlling, at least partly in parallel, processes in multiple vessels. For example, the process control device may be capable of managing processes in 12 vessels, 24 vessels, or 48 vessels. Each vessel may be a bioreactor. The vessels may be microscale vessels. In particular, the vessels may have a volume of less than 5 L, less than 1 L, or less than 500 ml. More particularly, the vessels may have a volume of between 1 ml and 500 ml.

Data analysis from experiments involving a process control device may involve multiple technicians spending many hours or days analyzing data in different data formats, possibly using a spreadsheet program such as Microsoft Excel. Results of the analysis may be inconsistent and/or subjective. In particular, it may be difficult to evaluate time series data of process parameters. Further, product quality attributes may be conflicting (e.g., inversely correlated), such that it is not possible to optimize two product quality attributes for the same process.

Moreover, it is common according to conventional approaches to set hard limits for product quality attributes. It may be difficult to determine which product quality attributes to set hard limits for and what those hard limits should be. Incorrectly setting hard limits (i.e., limiting values leading to the exclusion of a set of candidate cells) may cause the best performing set of candidate cells to be overlooked or unnecessarily excluded. Incorrect cell selection (i.e., selection of the wrong set of target cells) may lead to poor performance at larger scales or complications downstream (i.e., complications later in the manufacturing process). Accordingly, incorrect selection of target cells may lead to a waste of time, money, and resources. In particular, resources may be poorly allocated to a process using inefficient target cells, or too many iterations of the process for selection of the target cells.

Another problem is that data collected from a plurality of processes, particularly when the data is received at a process control device (e.g., some data is generated by the device itself and other data is generated by external analytic devices), is the presence of missing or unreliable data points. Such missing or unreliable data may lead to candidate cells being incorrectly evaluated and/or selection of an inefficient set of target cells.

Conventionally, data collected from a plurality of processes may be stored in multiple or many data files. The data files may be spreadsheet files, e.g., Microsoft Excel files. Occasionally, macros are used to help sort and graph the data. It may be challenging to determine how to use all the data, particularly time series data. It may be challenging to determine which process outputs to consider, or what characteristics the process outputs should have. Further, when the originally selected characteristics cannot be met by any set of candidate cells, it may be challenging to determine a new set of characteristics in order to arrive at a selection of target cells. In addition, it may be a problem to prioritize process outputs without discarding one in favor of another. It may also be a problem to minimize the number of iterations of the process of selecting the set of target cells.

According to an aspect, a computer implemented method for selecting at least one set of target cells from multiple sets of candidate cells is provided. The method comprises receiving data collected from a plurality of processes, wherein each of the processes produces a distinct set of candidate cells. The received data includes values of process parameters and process outputs of the processes, each of the process outputs being a product quality attribute or a key performance indicator for selecting the target cells. The method further comprises correlating the received data, as well as receiving a selection of the process parameters and a selection of the process outputs. The method further comprises receiving multivariate evaluation criteria for the selected process parameters and/or the selected process outputs, the multivariate evaluation criteria including one or more of the following:

- weights for prioritization,
- prioritization ranges and/or (prioritization) targets.

Each prioritization range may specify an allowable span of values, e.g., 3 to 10. The prioritization range may also include an extremum, e.g., to indicate whether a higher or lower value within the range is preferred.

Each prioritization target is an extremum (maximum or minimum), and/or a target value. For example, a prioritization target may include an extremum (e.g., maximum) and a target value (e.g., 4.5). Alternatively, the prioritization target may include an extremum without a target value, or a target value without an extremum.

"Each of the processes produces a distinct set of candidate cells" may be understood to mean that each one of the processes produces a different set of candidate cells. For example, if there are 18 processes, then 18 different sets of candidate cells are produced. Each set of candidate cells may be unique with respect to the other sets of candidate cells.

The term "process output" may be considered an umbrella expression that covers both product quality attribute and key performance indicator. In some cases, the term "variable" may be used to refer to either a process output or a process parameter.

A set of cells may be a group or collection of cells having the same type (e.g., a group of CS1_1 clones may be a set of cells).

A set of cells may be identified by a name (e.g., unique identifier) or location (e.g., culture station and/or vessel number) in a process control device. For example, "CS1" may be used to refer to cells located in a vessel in culture station 1 of the process control device. The digits following the "_" may identify individual vessels. For example, "CS1_1" may refer to the first vessel in culture station 1 and CS2_3 may refer to the third vessel in culture station 2.

The method further comprises calculating, via a multivariate selection function, scores for each one of the sets of candidate cells from the correlated data according to the multivariate evaluation criteria. The method further comprises ranking the sets of candidate cells according to the scores, and selecting at least one of the sets of candidate cells as the target cells using the ranking.

The target cells may be for use in (or to host) a chemical, pharmaceutical, biopharmaceutical and/or biological product. More particularly, the target cells may host the product, or the target cells may be the product. Each of the processes may be carried out by a process control device. The processes may be controlled by a single process control device, such that the processes are performed at least partly or entirely in parallel, or the processes may be carried out by additional process control devices. The processes may be carried out on a microscale. Alternatively, a portion of the processes may be carried out on a microscale and a portion of the processes may be carried out a macroscale. As another alternative, all the processes may be carried out on a macroscale. In this context, microscale may refer to vessels with a volume (i.e., working volume) as discussed above. Macroscale may refer to vessels having a volume (i.e., working volume) of greater than 1 L. More particularly, the microscale may be less than 250 ml and the macroscale may be greater than 3 L.

The process parameters may include set points, flow rates, feed characteristics, initial conditions, online measurements, and offline measurements. Examples of set points include temperature, pH, dissolved oxygen, stirring speed. Flow rates may include air flow, carbon dioxide, oxygen, and acids/base. Nutrient characteristics may include an initial nutrient feed day, nutrient feed volume, and feed additions. Initial conditions may include seeding density and osmolarity. Online measurements may include temperature, pH, dissolved oxygen, volume of fluid in the vessel in which the process is being carried out. Offline measurements may include glucose, lactate, viable cell density (VCD), amino acid levels, monoclonal antibody concentration.

A process output may be determined at the end of a corresponding process. For example, a process parameter may be cell viability (e.g., measured during the process) and a process output may be the final cell viability (at the end of the process).

Process outputs may include one or more of the following: a total quantity of cells, quantity of cells of per unit volume of input fluid, a chemical composition of the cells, a purity, amount of cell debris, amount of shear damage or chemical damage, starting material cost, energy cost for the process, product concentration, specific productivity, a profile describing the corresponding set of candidate cells (e.g., a glycan profile, a spectral profile), cell viability.

In one example, selected process outputs and corresponding prioritization targets may be specified as follows:

- final product concentration having a corresponding prioritization target to maximize, the target including a target value;
- final specific productivity having a corresponding prioritization target to maximize, the target having a target value;
- a profile distance from a profile of a corresponding set of candidate cells to a specified glycan profile or a specified spectral profile having a corresponding target of a minimum distance;
- final cell viability having a corresponding prioritization target to maximize, the target having a target value.

In the case of the profile distance, the process output is the profile of the set of candidate cells and the distance is an evaluation criterion received for the process output.

The cells (i.e., the target cells or the candidate cells) may be at least one of the following: a cell line, a cell strain, a clone. The multiple sets of candidate cells may form a heterogeneous pool of cells. More particularly, the multiple sets of candidate cells may form a heterogeneous transfection pool.

The values of the process parameters may include time series values. The process parameters may have been controlled (controlled process parameters) and/or measured (measured process parameters) during each of the processes. Measurements may have been carried out online, at line, or offline.

The terms offline, atline and online may refer to the frequency at which fluid in a vessel (e.g., bioreactor) is monitored, e.g., by performing monitoring steps such as sampling the fluid. The fluid may contain a set of candidate cells. The term offline may also indicate that analysis of monitoring results is, at least in part, performed in a laboratory. For example, a sample obtained via offline monitoring may be transferred to a laboratory for time delayed laboratory analysis. Offline measurements may be carried out less than once per hour, e.g., twice per day.

Atline measurements may be performed at a frequency similar to offline measurements. Atline measurements may involve analyzing an extracted sample in closer proximity to the vessel in comparison to offline measurements.

Online measurements may be carried out with greater frequency than atline or offline measurements. For example, online measurements may be performed more than once per hour, more than three times per hour, or about sixty times per hour. Online measurements may be carried out in-situ or ex-situ. In-situ measurements might not involve removing a sample from the vessel. Instead, a sensor (e.g., temperature or pH sensor spot) may be directly inserted into the vessel or separated from the vessel by a wall. Another possible in-situ configuration involves a sampling loop with one online sensor, or a non-destructive online analyzer and return of a sample to the vessel after analysis. In online ex-situ measurements, the sample may be transported to an online analyzer and does not return to the vessel after analysis.

In addition to selecting at least one set of target cells from multiple sets of candidate cells, the described approach may be used to select media for cell cultivation or to set conditions of the process.

The received data may include substantially all data from each of the processes. In particular, the received data may include values for each controlled process parameter and values for each measured process parameter. For example, if temperature within a vessel is measured throughout the process, then the received data may include all temperature measurements collected during the course of the process.

The method may further comprise identifying whether the received data for any one of the processes is incomplete, wherein one of the processes is identified as having incomplete data when data is not collected during a portion of the process. When any of the processes has incomplete data, the method may further comprise predicting values for the incomplete data using at least one multivariate technique. The multivariate technique may include partial least squares regression or interpolation. In addition, mechanistic modeling may also be used.

In some cases, the correlating may include verifying and correcting values of the data. The correcting may comprise revising or excluding values that violate one or more known metabolic dependencies. The dependencies may be ratios.

The method may further comprise applying mechanistic modeling to the received data to obtain additional values of the process parameters and/or additional process outputs. The method may further comprise supplementing the received data with the additional values of the process parameters and/or the additional process outputs.

More specifically, at least one mechanistic model (i.e., kinetic model) may be used to fit process parameter values and process outputs (possibly in the form of a process trajectory or cell growth profile) to monod growth kinetics. The mechanistic model may be used for data smoothing and/or for filling in missing data, as well as other beneficial applications. For example, given an example kinetic growth model, the maximum growth rate, death rate and other influences on growth can be determined from selected process outputs forming a process trajectory or cell growth profile. The mechanistic model and estimated values from the mechanistic model can be used to smooth process parameter values and process outputs. Smoothing may involve identifying and excluding measurement noise (i.e., inaccurate measurements). For example, viable cell density measurements may have an error of +/- 10%. The smoothing may involve excluding the erroneous measurements, e.g., process parameter values or process outputs.

In addition to smoothing and filling in of missing data, this approach provides meaningful information in explicit identification of growth, inhibition and death rates and can be used to calculate process outputs, such as maximum viable cell density or metabolite concentration (e.g., protein titer).

The method may further comprise excluding, from the correlated data, data received from ones of the processes according to exclusion criteria. If at least one of the selected process outputs has a corresponding acceptability range, then the exclusion criteria may include the corresponding acceptability range for the at least one of the selected process outputs.

The evaluation criteria may further comprise:

- a time based profile of one or more of the process parameters,
- a profile describing one or more of the process outputs,
- a trajectory describing time based development of one or more of the process parameters.

The profile describing the process outputs may be a glycan profile or a spectral profile. The spectral profile may be a spectral line.

The evaluation criteria may include a specified profile (e.g., a specified glycan profile and/or a specified spectral profile). The specified profile may correspond to (i.e., describe) a set of reference cells and may be used for comparison with the sets of candidate cells.

The evaluation criteria may be specific to a set of cells or groups of different cells.

The method may further comprise displaying the correlated data for the selected process parameters and/or the selected process outputs, comprising, displaying correlation patterns for the glycan profiles of the sets of candidate cells. Displaying the correlation pattern for the glycan profiles may involve displaying some or all final glycan measurements for one set of candidate cells and/or combining (e.g., via principal component analysis) glycan profiles for multiple sets of candidate cells.

The selection function may include an objective function, particularly a cost function. The objective function may be used to calculate a distance (e.g., a Euclidian distance) between different sets of cells. The distance may be calculated based on orthogonal components derived from the selected process parameters and selected process variables. For example, the objective function may provide a distance between one of the sets of candidate cells and a set of target values, e.g., from a set of reference cells. In other words, the output of the objective function may reflect the difference between the one of the sets of candidate cells and the set of target values. The output of the objective function may reflect a combination of distances according to the selected process parameter values and selected process outputs for the one of the sets of candidate cells and the set of target values (e.g., reference cells).

The selection function may include (possibly in addition to the objective function) at least one magnifying function (also referred to as a penalty function). The magnifying function may magnify a distance between values (e.g., between values associated with one of the sets of candidate cells and target values). Each of the prioritization ranges and/or targets may have an associated magnifying function. The magnifying function may be non-linear a nonlinear polynomial function, e.g., exponential or quadratic. In some cases, a logarithmic function (e.g., a function of the natural logarithm or Euler's number) may be used. The magnifying functions may be the same for all prioritization targets or ranges. This may have the advantage of making it easier to prioritize targets or ranges by weight.

Alternatively, magnifying functions may differ depending on the prioritization target or range. In particular, different magnifying functions may be used depending on the importance of the corresponding target.

Accordingly, an initial distance may be calculated using the objective function and then magnified using the magnifying function. After magnification, the distance may be modified via a weight. In particular, the distance may be multiplied by the weight.

Use of the magnifying function and the weights provides additional flexibility, however, either one or both may be omitted for particular prioritization ranges/targets or entirely omitted from the evaluation. In particular, it may be possible to provide optimal or at least adequate cell selection without use of the magnifying function and/or the weights.

Outputs of the selection function for each of the selected process parameters and/or the selected process outputs may be combined (e.g., summed) to calculate the score for the set of candidate cells.

In some cases, there are at least 5 sets of candidate cells, at least 10 sets of candidate cells, at least 20 sets of candidate cells, at least 30 sets of candidate cells, or at least 50 sets of candidate cells. Accordingly, there may be between about 5 and about 500, preferably between about 5 and about 200, sets of candidate cells.

According to an aspect, a computer program comprising computer readable instructions is provided. The instructions, when loaded and executed are on a computer system, cause the computer system to perform operations according to the method described above. The computer program may be implemented in the form of a computer program product, possibly (tangibly) embodied in a computer readable medium.

According to another aspect, a process control device for selecting at least one set of targets cells from multiple sets of candidate cells is provided. The device comprises a plurality of vessels, each of the vessels being configured to contain fluid including one of the sets of candidate cells. The device further comprises a robot capable of addressing each of the vessels, dispensing fluid to each of the vessels, and extracting samples of fluid form each of the vessels. The device also comprises a controller operable to control, at least partly in parallel, conditions in each of the vessels. The controller is further operable to receive data collected from a plurality of processes, wherein each of the processes produces a distinct set of candidate cells. The received data includes values of process parameters and process outputs of the processes, each of the process outputs being a product quality attribute or a key performance indicator for selecting the target cells. The controller is further operable to correlate the received data, as well as to receive a selection of the process parameters, and a selection of the process outputs. The controller is further operable to receive multivariate evaluation criteria for the selected process parameters and/or the selected process outputs. The multivariate evaluation criteria include one or more of the following:

- weights for prioritization,
- prioritization ranges and/or targets, wherein each target is an extremum (maximum or minimum) and/or a target value.

The controller is further operable to calculate, via a multivariate selection function, scores for each one of the sets of candidate cells from the correlated data according to the multivariate evaluation criteria. The controller is further operable to rank the sets of candidate cells according to the scores, and select at least one of the sets of candidate cells as the target cells using the ranking.

For example, only one of the sets of candidate cells may be selected as the target cells. Alternatively, multiple sets of candidate cells (e.g., 2-5) may be selected as target cells using the ranking.

Each of the vessels may have at least one of the following characteristics:

- it is a bioreactor or a microbioreactor,
- it includes stirring means for stirring its contents, wherein the stirring means may be an impeller (i.e., agitator),
- it includes delivery means for gas delivery, wherein the delivery means may be a sparge tube,
- it includes sensing means (e.g., one or more sensors) for measuring at least one of the following: pH, dissolved oxygen, temperature;
- it has a volume of: at least 1 ml, at least 10 ml, at least 15 ml, less than 2000 L, less than 1000 L, less than 100 L, less than 50 L, less than 5 L, less than 1 L;
- it is disposable.

The subject matter described in this application excludes treatment of the human or animal body by surgery or therapy, and diagnostic methods practiced on the human or animal body.

The subject matter described in this application can be implemented as a method or on a device, possibly in the form of one or more computer programs (e.g., computer program products). Such computer programs may cause a data processing apparatus to perform one or more operations described in the application.

The subject matter described in the application can be implemented in a data signal or on a machine readable medium, where the medium is (tangibly) embodied in one or more information carriers, such as a CD-ROM, a DVD-ROM, a semiconductor memory, or a hard disk.

In addition, the subject matter described in the application can be implemented as a system including a processor, and a memory coupled to the processor. The memory may encode one or more programs to cause the processor to perform one or more of the methods described in the application. Further subject matter described in the application can be implemented using various machines.

## Brief description of the drawings

Fig. 1 shows steps that may be performed in a method for selecting at least one set of target cells from multiple sets of candidate cells.

Fig. 2 shows an output of the method for selecting at least one set of target cells from multiple sets of candidate cells in which the set of target cells is highlighted.

Fig. 3 displays evaluations of multiple processes according to multivariate evaluation criteria.

Fig. 4 shows glycan profiles for multiple sets of candidate cells.

Fig. 5 displays multivariate evaluations of multiple sets of candidate cells.

Fig. 6 also shows steps that may be carried out as part of the method for selecting at least one set of target cells from multiple sets of candidate cells.

Fig. 7 shows an exemplary use of mechanistic modeling to smooth process outputs.

Fig. 8 is a perspective view from above of a portion of a process control device.

Fig. 9 shows a cross-sectional view of a vessel of the process control device.

## Detailed description

In the following text, a detailed description of examples will be given with reference to the drawings. It should be understood that various modifications to the examples may be made. In particular, one or more elements of one example may be combined and used in other examples to form new examples.

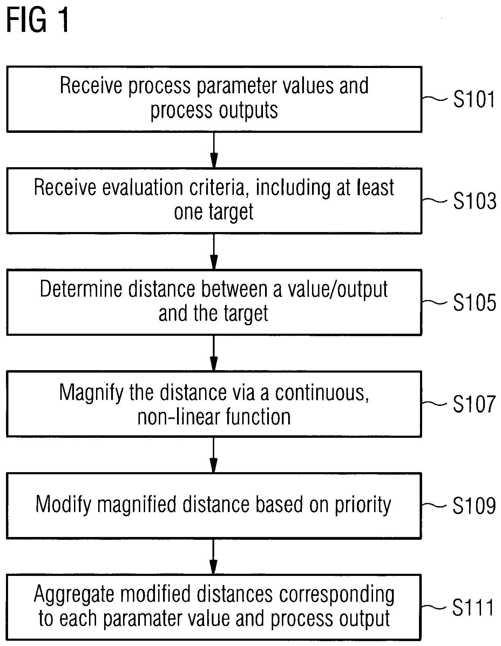

Fig. 1 shows steps that may be carried out in a method for selecting at least one set of target cells from multiple sets of candidate cells. At step S101, data collected from a plurality of processes is received. Each of the processes produces a distinct set of candidate cells. The received data includes values of process parameters and process outputs of the processes. Each of the process outputs is a product quality attribute or a key performance indicator for selecting the target cells.

In the following example, the sets of candidate cells are clones and the target cells are the best clone in the sets of candidate clones. It should be understood that although the following example is described in the context of clones, it is applicable to other types of cells.

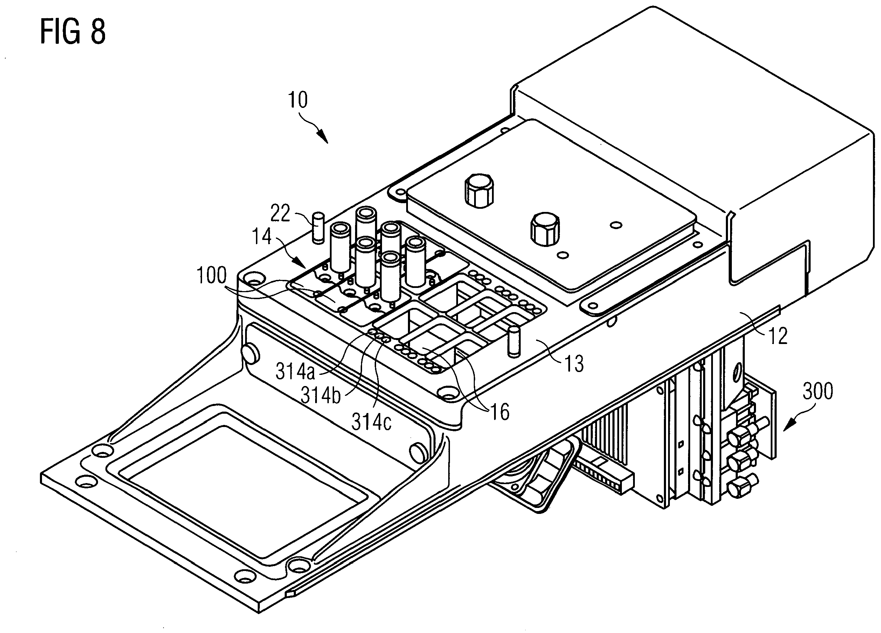

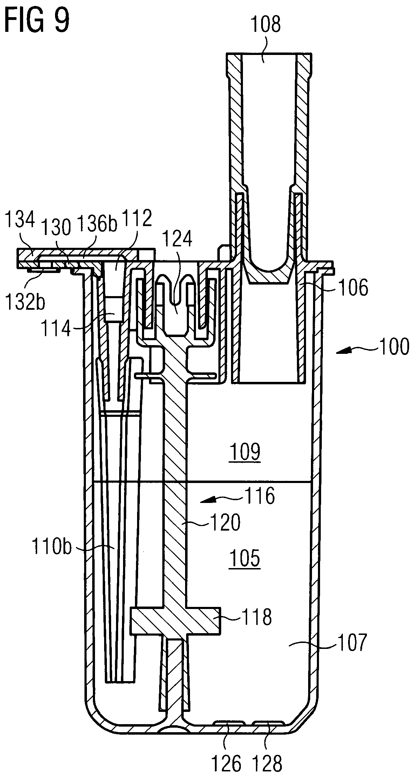

The plurality of processes are carried out in parallel via a process control device (shown in Figs. 8 and 9 and discussed in more detail below). More specifically, there are 24 processes carried out in 24 vessels. Each vessel has about 11 to 15 ml working volume. Data from offline measurements (e.g., glycan measurements, glucose, lactate, viable cell density (VCD), amino acid levels, monoclonal antibody concentration) is received at the process control device.

The received data is organized into two separate tables (not shown) for 24 candidate clones. A first table is a process data table for seven process parameters including cell viability, process concentration, and specific productivity. Process parameter values were obtained over the course of several days taking one measurement per day. The second data table is a quality data table. The quality data table includes process outputs, specifically, one glycan profile having 13 separate process outputs (i.e., measurements) for each clone and a calculated distance from a target profile.

Step S101 may also include correlating the received data. Step S101 may also include receiving a selection of the process parameters and a selection of the process outputs. In the present case, the selection may be represented by the data stored in the process data table (i.e., the selection of process parameters) and the quality data table (i.e., the selection of process outputs).

At step S103, multivariate evaluation criteria for the selected process parameters and/or the selected process outputs is received. According to the present example, the multivariate criteria includes the following four prioritization targets:

- final product concentration: maximize, but at least minimum product concentration value (e.g. 3.5 g/L);
- final specific productivity: maximize, but at least minimum specific productivity value (e.g. 4.5 grams per cell per day);
- quality (distance from profile of candidate cells to profile of reference cells): minimize, but not more than maximum distance value (e.g. 15 units);
- final viability: maximize, but at least minimum viability value (e.g. 65%).

The distance specified for the quality prioritization target may be a Euclidian distance, calculated according to the formula specified in the context of S105.

The prioritization targets may be listed in order of priority. Priorities may be set by weights, as discussed in more detail below. For example, the final product concentration may have a higher weight than the quality.

In the example above, each of the prioritization targets includes an extremum; three of the prioritization targets include a maximum and one prioritization target includes a minimum. Each of the prioritization targets also includes a target value (particularly the minimum product concentration value, the minimum specific productivity value, the maximum distance value and/or the minimum viability value, e.g., 3.5 g/L, 4.5 grams per cell per day, 65% or 15 units, as specified above, respectively).

It should be noted that although the example above only includes prioritization targets; prioritization ranges may be supplied as an addition or alternative.

The example is carried out in the process control device described above. Accordingly, when the four prioritization targets are applied to the 24 candidate clones, no clone meets all criteria. However, lowering the limit for final viability (i.e. the minimum viability value) from 65% to 60% leads to the result that a clone referred to as CS1_7 is selected as the target clone. CS1_7 has a product concentration of 4.5 g/L, a specific productivity of 4.51 grams per cell per day, a quality of 14 and a final viability of 60%. Clone CS1_7 would be selected as the target clone according to conventional approaches, particularly because conventional approaches typically rely on at least one hard limit that leads to the exclusion of clones that do not meet the hard limit.

Conventional approaches including hard limits can be automated using a decision tree. Further, multiple hard limits can be set by the user. However, use of one or more hard limits may lead to strict exclusion of clones that do not meet those limits and might not result in the selection of the best (i.e., optimal) target clone.

Applying a multivariate approach, as discussed in more detail in the following steps, leads to selection of a clone that better matches the given criteria, increasing the likelihood that the selected clone will lead to a safe and effective product. The clone selected according to the following steps would have been excluded according to the traditional approach because the specific productivity of the clone falls just outside the specified limit of 4.5 grams per cell per day. The steps discussed below enable process parameter values and process outputs to be combined into a common score. Scores for candidate clones can then be sorted to provide a final ranking leading to the selection of a given number of clones. A multivariate selection function (discussed in more detail below) used in the ranking accounts for each prioritization target and/or prioritization range, as well as weights for prioritization. Accordingly, a consistently ranked list of available clones is provided. Subjectivity is excluded and each ranking performed on the same data (even by different users) leads to a reliable and consistent result.

In steps S105 to S111, a multivariate selection function is used to calculate scores for each one of the sets of clones from the correlated data according to the multivariate evaluation criteria.

At step S105, an objective function may be used to determine a distance between a process variable value (process parameter value and/or process output) and the prioritization target. More particularly, multiple process parameters and/or process outputs may be combined into a component, and the distance may be between the component and the prioritization target. The component may be derived using principle component analysis (PCA), however, other means suitable for calculating orthogonal components (i.e., vectors) from variables could also be used. Accordingly, components for each of the prioritization targets may be calculated. The components may be orthogonal (i.e., not correlated) and suitable for Euclidean distance calculations. Distance calculations may also be performed using partial least squares or orthogonal partial least square projections. According to one example, the distance between a candidate clone and the prioritization target may be calculated as follows:

1. calculate projection vectors $t_i$ for each clone,
2. the projection vectors $t_i$ are orthogonal, with a length proportional to the variance explained by the vector, and

3. the distance (D) between specific clones, $D^2 = \sum_{k=0}^{i} \left( t(c,i) - t(r,i) \right)^2$

- where c is a candidate clone, and
- r is the prioritization target(s) or set of reference clones.

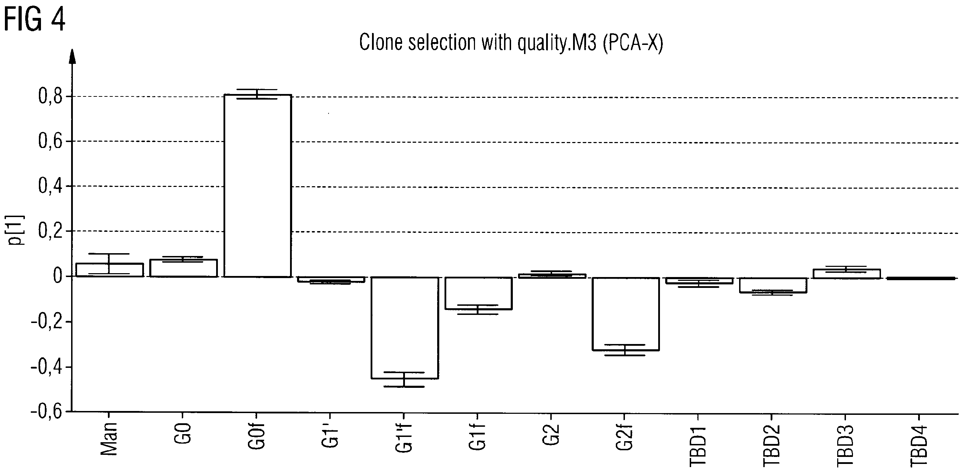

Thus, an exemplary objective function is provided in point 3 above. The objective function may include principal components (i.e., projection vectors) derived from process parameters and/or process outputs. The objective function may include a Euclidean distance calculation involving the principal (orthogonal) components. The combination of Euclidian distance and orthogonal components may be particularly advantageous, since possible correlations between variables (e.g., correlated glycans, as shown in Fig. 4) are reflected in the orthogonal components and an assumption that the variables are not correlated is unnecessary.

Regarding points 1 and 2 above, conventional approaches may consider a subset of process outputs as a basis for evaluation. In such approaches, prioritization may assume that there is no correlation between these process outputs, particularly because it is difficult to prioritize one variable over another variable when the variables are correlated. By comparison, the objective function in point 3 does not require an assumption that the variables (i.e., parameters or outputs) are not correlated, since orthogonal projection vectors reflecting variable correlations are calculated from the variables and then used to determine the distance D.

Advantageously, the objective function may consider all process parameters and process outputs (i.e., if all are selected) or a subset (e.g., proper subset) of the process parameters and process outputs, as discussed in the example. Further, the absence of hard limits may prevent exclusion of optimal (e.g., the most efficient) clones.

Returning to the example, the clone CS2_2 may have a product concentration of 4.8 g/L. Step S105 may include determining the distance between 4.8 g/L and the target product concentration of 3.5 g/L. Determining the distance may involve normalizing the distance. In this case, since the product concentration is to be maximized, the inverse of the difference between the product concentration of clone CS2_2 and the target product concentration may be used.

After the distance is determined via the objective function, the distance is magnified in step S107. In particular, the selection function includes a magnifying function. The magnifying function may be a continuous non-linear function.

Using a non-linear magnifying function (in contrast to a linear function) may be advantageous, since such a function will favor clones (i.e., cause them to be ranked higher) having more acceptable values (e.g., process outputs) in comparison to clones having fewer acceptable values. In this context, an acceptable value may be within a prioritization range (e.g., an acceptable value of 4.5 within a range of 2-6) or between a target value and its corresponding extremum (e.g., an acceptable value of 4.5 having a target value of 3 and an extremum of maximize).

In contrast, use of a linear magnifying function (or no magnifying function) may result in clones that meet only a few of the targets (e.g., having relatively few acceptable values in between a target value and its corresponding extremum) being selected, if the few acceptable values (e.g., process outputs) are sufficiently close to the extremum in comparison to other clones. In other words, use of a linear function (or no function) could cause selection of clones that do not have acceptable values with respect to a relatively large number of targets. This may be undesirable.

The magnifying function may also be referred to as a penalty function, since the magnifying function serves to increase the impact of the distance (i.e., impose a penalty according to the distance) between a value (e.g., process output) corresponding to a candidate clone and the prioritization target.

The magnifying function may be the same for all prioritization targets. In this way, the magnifying function can be used to influence values corresponding to a candidate clone (without consideration of others), while the weights can be used to prioritize values for corresponding to different prioritization targets against each other (e.g., by setting a weight for one prioritization target higher than a weight for another prioritization target).

At step S109, the magnified distance may be modified based on priority. In particular, the weights for prioritization may be used to modify the magnified distance. Each weight may be a value between 0 and 1 and the magnified distances may be modified by multiplying them by their corresponding weights.

At step S111, the modified distances may be aggregated to produce the score corresponding to the clone. In particular, for each clone, the distances for all variables (i.e., process parameters and process outputs) are combined into a total distance value. More specifically, the distances may be added together.

Applying this approach results in clone CS2_2 being ranked higher (i.e., having a lower total distance from the prioritization target) than clone CS1_7. In particular, clone CS2_2 has a product concentration of 4.8 g/L, a specific productivity of 4.45 grams per cell per day, a quality of 8.0 and a final viability of 70%. Even though the specific productivity target of 4.5 grams per cell per day is not reached, the described approach results in the selection of clone CS2_2 as the target clone. Although clone CS2_2 has better values in targets other than specific productivity in comparison to the other candidate clones, clone CS2_2 would still have been discarded according to conventional approaches.

Use of the selection function and the multivariate approach described above ensures the best possible ranking and enables consideration of an arbitrary number of process parameters and process outputs in clone selection.

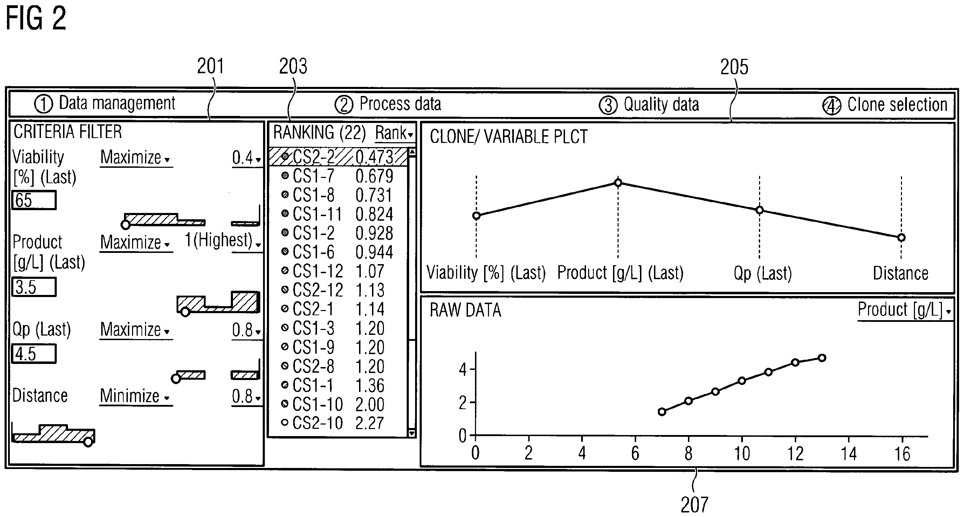

Fig. 2 shows the selection of a set of target cells from multiple sets of candidate cells. In the example of Fig. 2, the target cells and the candidate cells are clones, however, the described approach is applicable to other types of cells.

In a criteria filter pane 201, multivariate evaluation criteria for the selected process outputs are shown. In particular, the displayed prioritization targets include final viability (shown as a percentage with a target value of 65), final product concentration (shown in g/L with a target value of 3.5), final specific productivity (shown as "Qp", with a target value of 4.5), and quality (shown as "Distance", with a target value of 15 units). The final viability, product concentration, and final specific productivity are to be maximized. The quality (i.e., distance) is to be minimized. Weights for prioritization are also shown, with a weight of 0.4 for final viability, a weight of 1 for final product concentration, a weight of 0.8 for final specific productivity, and a weight of 0.8 for quality.

A ranking pane 203 is also shown. The ranking pane 203 includes a first column for the candidate clones and a second column for the corresponding scores of the candidate clones. As shown in the depicted example, the clone CS2_2 (displayed as CS2-2) has the lowest score of 0.473 and therefore the highest rank. A clone/variable plot 205 shows each of the criteria for clone CS2_2 in relation to the other candidate clones. Thus, clone CS2_2 had the highest product concentration, the final viability and final specific productivity values of CS2_2 were about average, and CS2_2 had a relatively low quality (i.e., distance). However, particularly because of the weights for prioritization allocated to each of the criteria, CS2_2 was given the highest rank.

A raw data pane 207 shows a process trajectory for CS2_2 in comparison to process trajectories for the other clones.

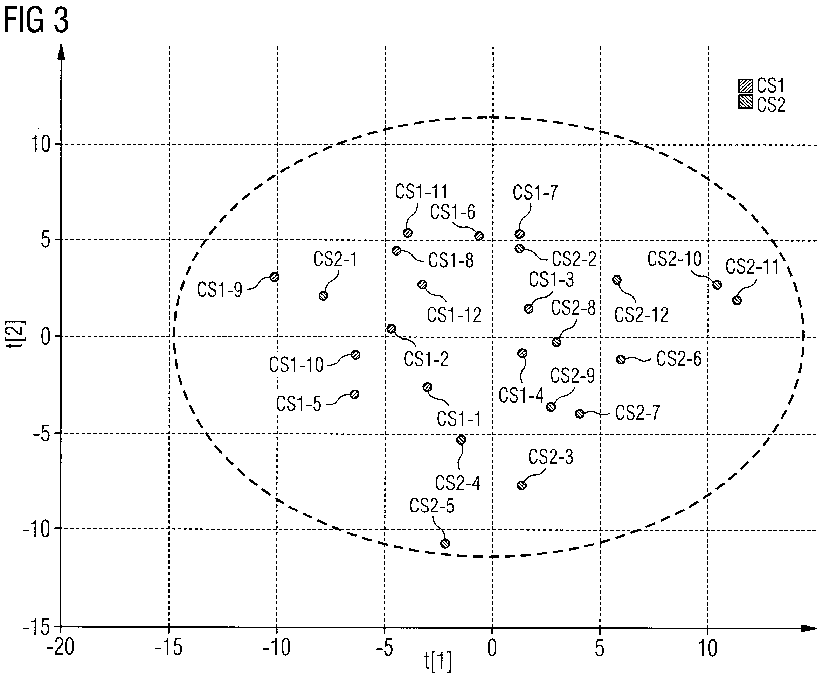

Fig. 3 shows how process parameter values and process outputs can be combined for evaluation. In particular, multivariate statistical techniques (e.g., principal component analysis) may be used to combine multiple process parameters and/or multiple process output. For example, cell similarity indices may be calculated in both the quality domain e.g., using glycan profiles or spectral fingerprints, as well as in the process domain, where time series data can be combined and evaluated. Process outputs may correspond to the quality domain, whereas process parameters may correspond to the process data domain.

Further details regarding principal component analysis and other multivariate statistical process control methods may be found in "Process Analysis, Monitoring and Diagnosis Using Multivariate Projection Methods", Theodore Curti, John F. McGregor, Chemometrics and Intelligent Laboratory Systems 28, 1995, which is incorporated herein by reference.

In addition to principal component analysis, partial least squares and/or orthogonal partial least squares may also be implemented for selecting target cells. Accordingly, an overview map may be presented, as shown in Fig. 3, such that similarities between cells can be visualized and interpreted.

In the example depicted in Fig. 3, a CS1 group of clones and a CS2 group of clones are displayed. The groups CS1 and CS2 may be delineated according to separate culture stations of the process control device. Although clones are referred to, the disclosed techniques are applicable to other types of cells.

In the depicted example, each point is the multivariate representation of the process parameters and the process outputs for one clone. The combination of process parameters and process outputs, as shown in Fig. 3, may highlight potential experimental problems not easily detected in the underlying data. Further, the depicted multivariate analysis may be useful in selecting the target clone.

Multivariate statistics (e.g., principal component analysis) may be particularly useful when clones are represented using a set of process outputs (e.g., a glycan profile). Accordingly, the multivariate statistical analysis may provide similarity indices (e.g., principal components derived from process outputs), as shown in Fig. 3, to be used in the ranking of sets of candidate clones. The similarity indices may be evaluated using the objective function, e.g., as discussed in the context of step S105 above.

Further, the depicted example may also provide information on the correlation among different process parameters. This information can be applied when evaluating process parameters and process outputs against the prioritization targets, thereby improving the ranking. Further, correlation information may be useful for the user when setting values for prioritization targets or prioritization ranges.

In the example of Fig. 3, data is collected from 24 processes. Each of the processes produces a distinct clone. The CS1 clones were produced in a first culture station and the CS2 clones were produced in a second culture station. Data collected from the processes carried out to produce the clones may be correlated and further evaluated as described in the context of Fig. 1. Fig. 3 shows values with respect to two principal components, t[1] and t[2]. In order to calculate scores via the multivariate selection function further principal components may also be evaluated in addition to prioritization ranges, prioritization targets, and weights for prioritization as discussed in the context of Fig. 1.

The orthogonal components used in Fig. 3 may be more efficient for use in the ranking process in comparison to the many process parameters and process outputs. This is particularly the case because of conflicting (e.g., inversely correlated) targets, as discussed in more detail in the context of Fig. 4.

Fig. 4 shows process outputs (i.e., glycans) collected from a plurality of processes. More particularly, Fig. 4 shows a glycan profile derived from data collected from processes that produced the components of the 24 clones shown in Fig. 3.

In the context of Fig. 4, targets (i.e., prioritization targets) have been set for both G1'f and G0f. The prioritization targets include an extremum, i.e., a minimum. Accordingly, it is desirable for both G1'f and G0f to have values as low as possible. However, since G1'f and G0f are inversely correlated, it is not possible to minimize both G1'f and G0f. Accordingly, one of these prioritization targets must be prioritized over the other. In other words, either the prioritization target of minimizing G1'f must be prioritized over the prioritization target of minimizing G0f, or the prioritization target of minimizing G0f must be prioritized over the prioritization target of minimizing G1'f. More generally, once multivariate evaluation criteria for the selected process outputs are received, a display may be provided indicating an inverse correlation between at least two of these selected process outputs. Accordingly, an external input (such as by the user) may provide further prioritization targets before the scores are calculated via the multivariate selection function.

In the example depicted in Fig. 4, the glycan profile for each clone is based on 12 process outputs. The glycan profile provides information regarding dependencies between different process outputs. In particular, each glycan may be considered a distinct process output and the glycan profile may show a correlation pattern among the glycans.

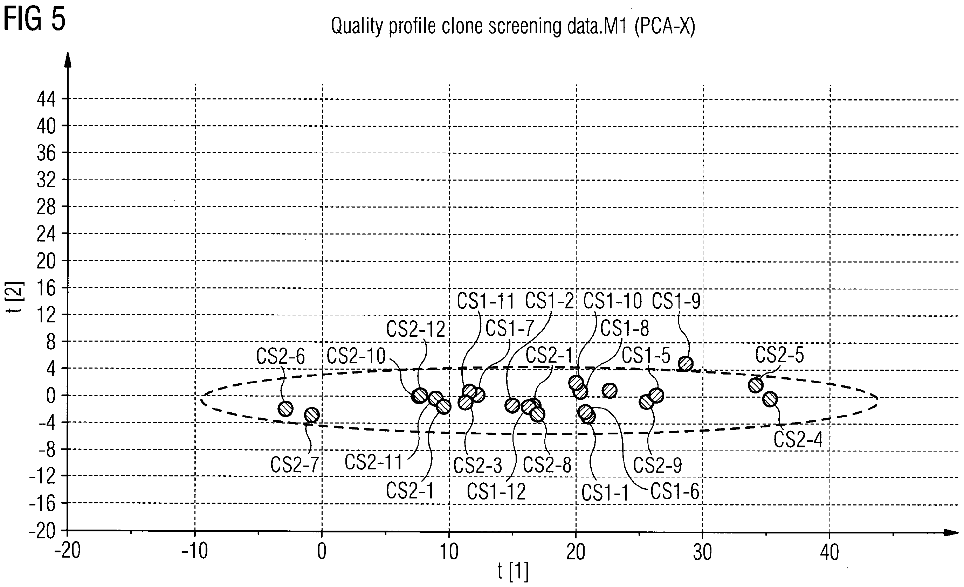

Fig. 5 shows a projection plot depicting a principal component transformation of the 12 glycan profile variables depicted in Fig. 4. Accordingly, a value for each clone is displayed and the value is derived from the 12 glycan profile variable values for that clone. The principal components depicted in Fig. 5 are an example of components (i.e., projection vectors $t_i$) that can be used in the context of the objective function discussed in connection with Fig. 1. Further, the components depicted in Fig. 5 may be used to calculate the scores via the multivariate selection function.

The x axis depicted in Fig. 5 represents 98% of the total variation in the 12 glycan profile variables. Thus, 98% of the variation in the 12 glycan profile process outputs is one-dimensional. While combining process outputs into principal components may make calculation of the scores via the multivariate selection function more efficient, it would also be possible to use process parameters and/or process outputs directly, without the additional step of determining principal components.

It should be noted that even though principal component analysis is discussed in the context of Figs. 3 and 5, other multivariate statistical analysis techniques may also be used, such as partial least squares regression and/or orthogonal partial least squares regression.

In the example of Fig. 5, the origin of the projection plot is a score corresponding to a reference clone. The reference clone may be represented by a plurality of target values.

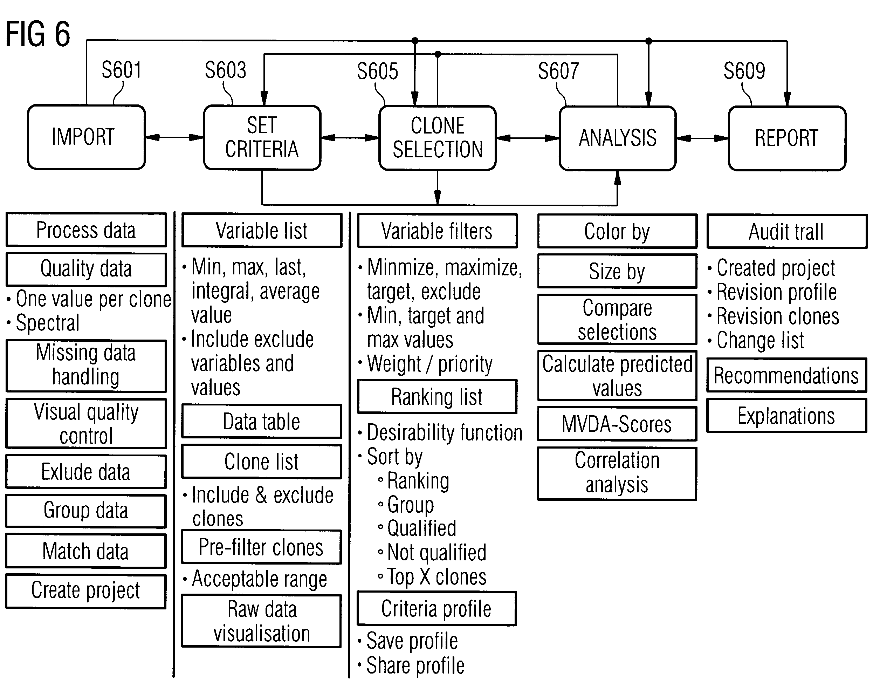

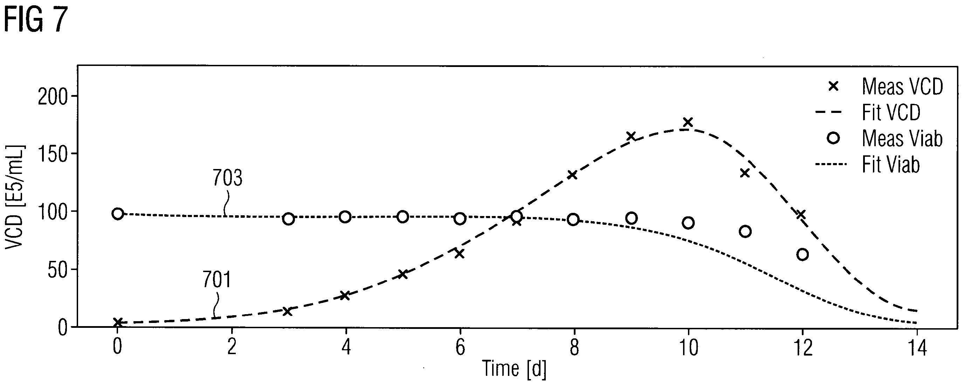

Fig. 6 shows functionality of a tool that may be used to implement the method for selecting at least one set of target cells from multiple sets of candidate cells, as discussed above. The tool may be implemented in hardware and/or software. In particular, the tool may be implemented in the process control device mentioned above and depicted in Figs. 7 and 8.

As shown by the arrows in Fig. 6, there may be some overlap between steps S601 to S609. For example, actions carried out in step S601 may be carried out after actions carried out in step S603. Similarly, as indicated by the arrows, some of the results of step S601 may be used in steps subsequent to step S603, without the processing carried out in step S603. Corresponding considerations may apply to the other steps.

At step S601, data is imported. Step S601 may include receiving data collected from a plurality of processes, wherein each process produces a distinct set of candidate cells. The imported data may include process data. Process data may be considered synonymous with process parameter values. Process data may include time dependent data sampled from the processes. Examples of process data are pH, product titer, viable cell density, glucose, dissolved oxygen, and/or oxygen consumption.

In addition, in step S601, quality data may be imported. Quality data may be understood as process outputs. The quality data may describe the end quality of the candidate cells. More particularly, the quality data may describe the cell line, cell strain, or clone processed. Typical quality data may be glycosylation patterns, charge variants, aggregates, low molecular weight species, and/or glycan residues displayed as a profile (e.g., as profile vectors). Process outputs may also include aggregated process data. For example, viable cell density may be measured throughout the process and the measurements may be received as process parameter values. A final viable cell density may be received as a process output of a process.

Step S601 may also include handling missing data. This step may be carried out in the context of correlating the received data. Missing data may include data that is not collected or sampled frequently enough. For example, glucose and/or lactate may be sampled only once per day, however, a more complete picture of glucose and/or lactate levels may be desired. Accordingly, it may be desirable to simulate hourly measurement of glucose or lactate by filing in data for missing samples.

The missing data may be filled in using mechanistic modeling procedures and/or multivariate prediction (e.g., partial least squares regression). Mechanistic modeling and multivariate prediction models may also be used to predict future behavior of candidate cells. Accordingly, mechanistic modeling may provide input on the biological state of candidate cells at any given time and may enable early evaluation of candidate cells.

Prediction of future behavior may make it possible to determine what would happen if processing of candidate cells is terminated prematurely, e.g., due to an infection. For example, if processing of candidate cells is terminated prematurely, prediction of future behavior of the candidate cells may still enable process data for those candidate cells to be incorporated into calculations performed via the multivariate selection function in order to arrive at a score for the candidate cells that can be used in the ranking discussed above.

Mechanistic modeling can also be used to improve the quality of measured data by verifying and correcting values that violate known metabolic ratios, thereby ensuring that higher quality data is used in the ranking. Mechanistic modeling may also be used to estimate process parameter values (e.g., cell death rate) that are difficult or impossible to measure directly and thereby add further viable information that can be used when calculating scores for each of the candidate cells.

For example, viable cell density measurements may have an error of +/- 10%. Smoothing of the measurements may be carried out in order to exclude the erroneous measurements, e.g., process parameter values or process outputs.

A specific example of a mechanistic model that can be used to fill in missing data or improve the quality of measured data (e.g., via data smoothing) is described in the context of Fig. 7.

Step S601 may also include visual quality control. Accordingly, the received data may be graphically presented such that outliers can be easily identified and excluded. Further, data can be corrected as desired.

Step S601 may also involve data exclusion. More particularly, at least one of the processes may fail. In particular, events such as contamination and/or system failure, unexplained or inconsistent biological factors, or human error may lead to failure of the process. Data from the failed process may be excluded.

Step S601 may also include grouping the received data. For example, replicates, minipools, or biosimilar cells (e.g., clones) may be grouped for analysis. Prioritization ranges and/or prioritization targets may be set for each group, or for the entire set of cells.

Step S601 may also include data matching. In particular, data may be received from various sources. The data may be matched and synchronized for analysis. The sources may include the process control device itself, and possibly an external analysis device. External analysis devices may include one or more of the following: a device for offline spectroscopy measurement (spectrometer), a device for inline (i.e., online, in situ) biomass measurement, a device for nutrient and metabolite measurement.

The spectrometer may be an apparatus to separate (subatomic) particles, atoms, and molecules by their mass, momentum, or energy). For example, spectroscopic measurements may be carried out using an Acquity iClass UPLC and Xevo TQS triple quadrupole mass spectrometer (Waters, Milford, MA). Other devices may also be used.

The device for nutrient and metabolite measurement may measure parameters online. Examples of nutrients include glucose and lactate. Examples of metabolites include methanol and ethanol. In particular, the device may perform up to 60 analyses per hour for a filtration probe and up to 30 analyses per hour via a dialysis setup. More specifically, glucose and lactate may be analyzed using a Bioprofile flex (Nova Biomedical Corporation, Waltham, MA).

Viable cell concentration may be analyzed using a cell viability analyzer, such as the Vi-Cell Automated cell viability analyzer (Beckman Coulter, Brea, California). Other devices may also be used.

Step S601 may also include creating a project. The project may provide a framework to use and store multivariate evaluation criteria, as discussed in more detail below.

At step S603, criteria for the selected process parameters and/or the selected process outputs may be set. In particular, step S603 may include selecting a (proper) subset of the process parameters and/or a (proper) subset of the process outputs. Accordingly, the selection of the process parameters may exclude one or more of the process parameters. The selection of the process outputs may exclude one or more of the process outputs. The selected process parameters and/or process outputs may then be received, e.g., stored by, the process control device.

Step S603 may include displaying the received data collected from the plurality of processes. Further, the process parameters and process outputs may be displayed.

Data may be displayed in the form of a table. In particular, process parameter values may be visualized in a data table. The table may facilitate correction of obvious errors in the data.

Display of the data in the data table may facilitate exclusion of candidate cells or identification of one or more sets of candidate cells as reference cells. For example, if one of the sets of candidate cells exhibits an abnormal profile, the set of candidate cells may be eliminated.

Acceptability ranges for process outputs may be set. The acceptability ranges may be set so as to exclude one or more outliers. Setting acceptability ranges (i.e., acceptable ranges) may be part of a prefiltering process. To aid in the setting of acceptability ranges, an overview display of the received data may be provided. Accordingly, it can be easily determined how much of the received data is excluded by setting an acceptability range. If the acceptability range is set too strictly, the number of sets of candidate cells that pass through the prefiltering process may be too limited. For example, if an acceptability range is set to filter out sets of candidate cells having a low titer, the absence of high titer producing sets of candidate cells among all the sets of candidate cells may limit the number of sets of candidate cells that are passed through the prefiltering process. This may be undesirable.

A display showing the sets of candidate cells that are excluded for a specified acceptability range may be useful in assisting the user to set a suitable acceptability range. Step S603 may also include a raw data visualization. The raw data visualization may help facilitate understanding of which data is excluded by the specified acceptability ranges.

Step S605 may include receiving multivariate evaluation criteria for the selected process parameters and/or the selected process outputs. The multivariate evaluation criteria may include aggregating or further processing at least one of the selected process parameters and/or the selected process outputs.

The multivariate evaluation criteria may include weights for prioritization. The multivariate evaluation criteria may include prioritization ranges and/or targets, wherein each target is an extremum and/or a target value. Prioritization targets may be set for a (proper) subset of the selected process parameters and/or process outputs. The extremum may be to maximize or minimize. The prioritization target may include a target value or set point. The target value may be a specific reference or limit value.

Prioritization ranges may be received as input from the user. However, the prioritization ranges may be modified when calculating the scores for each one of the sets of candidate cells. Weights for prioritization may be used to define the importance of each of the prioritization targets. For example, a weight of 1.0 may be given to the most important prioritization target. Weights close to 0 may be given to relatively unimportant prioritization targets or prioritization ranges.

Step S605 may include calculating, via the multivariate selection function, scores for each one of the sets of candidate cells from the correlated data according to the multivariate evaluation criteria. The multivariate selection function may include an objective function, more particularly a cost function. The objective function may be referred to as a desirability function. The objective function may be used to rank the candidate cells according to how well they fit the multivariate evaluation criteria. The objective function may be non-linear. The objective function may be exponential, e.g., quadratic. The objective function may quantify the distance from a numerical target (e.g., a prioritization target or range) and aggregate penalties based on the evaluation criteria and the weights for prioritization. The numerical target may be a set of reference cells having a biosimilar definition in comparison to the sets of candidate cells. The objective function may be as specified in the discussion of step S105 of Fig. 1 above.

Calculation of scores for each one of the sets of candidate cells may be performed multiple times (i.e., iteratively) using different evaluation criteria, particularly because it may be determined that two or more of the evaluation criteria are inversely correlated, as shown in Fig. 4.

The multivariate selection function may produce a score that reflects how well a set of candidate cells fulfills the multivariate evaluation criteria. The score may be referred to as a desirability index. Selected profiles for candidate cells can be visualized graphically and enable the inspection of cell profiles with scores that are close to each other, as shown in Fig. 2. For example, if a set of candidate cells is ranked fifth, e.g., in the ranking 203, the corresponding variable plots for the set of candidate cells and other sets of candidate cells may facilitate determination of how the ranking was generated. Such comparisons may enable reprioritization and guide further selection iterations. Accordingly, the display of graphical data, e.g., as shown in Fig. 2, may facilitate analysis that would not be possible from the underlying raw data. Moreover, the number of selection iterations may be reduced and require less user intervention in comparison to conventional approaches.

A multivariate correlation analysis tool, e.g., the tool implementing the method for selecting at least one set of target cells for multiple sets of candidate cells, can help the user to adjust the selection process, including prioritization targets, weights, and ranges, in order to ensure that the optimal set of target cells is selected. Further, the selection of sets of candidate cells discussed above may reflect both process parameter values and process outputs.

The evaluation criteria can be saved for later use with other sets of candidate cells or shared with other users. Use of the same multivariate evaluation criteria by multiple users for different evaluations may ensure consistency. In particular, the same criteria may be applied to different data sets by different users. This may eliminate the subjectivity that is often present in conventional approaches. Further, the same criteria may be used for different batches of data from the same project.

At step S607, analysis may be carried out. As noted above, some of the actions carried out in the context of step S607 may be carried out in combination with or even before steps carried out in the context of step S605.

To facilitate ranking of the sets of candidate cells, at least one of the prioritization targets may be calculated. More particularly, at least one of the target values may be calculated. For example, integrals and/or averages of the process parameter values, relations, and predictions based on process history may be calculated. Further, some process outputs may also be calculated. Calculated and/or predicted values may provide more stable input for the ranking of the sets of candidate cells and the selection of at least one of the sets of candidate cells as the target cells.

In addition, mechanistic modeling algorithms may be used to fill in missing data and predict process trajectories and/or final results, as discussed above. For example, candidate cells that stopped growing earlier than expected may be compared with other candidate cells that continued producing results until the end of their corresponding processes. Process data and quality data for the candidate cells that stopped growing earlier than expected may be extrapolated or interpolated from the other candidate cells that continued producing results until the end of their corresponding processes. The trajectory predictions for candidate cells that terminated early may provide a more complete set of data that can be used to improve the ranking of the sets of candidate cells.

Further, multivariate modeling, as discussed above in connection with correlating the received data, may be used to compensate for errors in measurement due to sampling, handling or inherent flaws in external analytic devices. The multivariate modeling may use fundamental bioprocessing correlations to produce more reliable data. This may be part of the correlation carried out after data is received or may be carried out in a further iteration of the described method after an initial ranking of the sets of candidate cells has been produced. The score calculated via the multivariate selection function may be based on all selected process parameters and all selected process outputs. Further, information from the process trajectory of each process may also be used. Scores calculated via the multivariate selection function may be compared to a prioritization target (e.g., biosimilar cells or a group value).

Comparisons can be made within a group or to a specific reference process (i.e., a process that produces reference cells). These comparisons may facilitate setting different prioritization ranges, targets and weights in further iterations of the above described method. Further, process trajectories for each of the sets of candidate cells can be used to calculate distances between sets of candidate cells, groups, or from target values for use in the ranking of the sets of candidate cells. In addition to process trajectories, other multivariate criteria may be used in the ranking of candidate cells, particularly other quality data such as glycan profiles.

Step S607 may also include correlation analysis, as discussed in connection with Figs. 4 and 5. The correlation analysis may give information on how to improve candidate cell selection, since some of the received evaluation criteria may contradict other criteria (e.g., the criteria may include inversely correlated targets). Understanding the correlation between different variables (i.e., process parameters and process outputs) may facilitate tuning of the tool in order to arrive at an optimal selection of target cells, e.g., in a minimal number of iterations.

Selected candidate cells may be compared with all other candidate cells for analysis. Multivariate approaches such as principal component analysis (PCA) and partial least squares (PLS) regression can be used for visual comparison and calculation of distance between different sets of candidate cells. Graphic representation and visualization of the results may help the user determine similarities between specific sets of candidate cells or groups. Components describing individual sets of candidate cells may be displayed as dots in two-dimensional diagrams, as shown in Figs. 3 and 5, with a possibility to color or set sizes according to any variable or preference used in the selection process. The coloring and sizing may be part of a three-dimensional graph.

At step S609 a report may optionally be produced. The report may include a document reporting one or more selection results, the multivariate evaluation criteria, prioritization targets, target values and/or extrema, rankings, statistical correlations, and/or observations. In particular, the report may provide information supporting (i.e., reasons for) the selection of the set of target cells from the multiple sets of candidate cells. If multiple iterations of the method have been carried out, the report may include a summary of results from each iteration.

Fig. 7 shows an example of how mechanistic modeling can be used to smooth process outputs. The example of Fig. 7 depicts "E5" cells, i.e., a particular type of candidate cell.

VCD measurements (in cells/mL) are denoted by "x" marks and cell viability measurements (indicating the percentage of cells that are viable, i.e., still alive) are denoted by filled in circles. The VCD measurements include an initial VCD measurement of 2.38827. The cell viability measurements include an initial cell viability measurement close to 100%. VCD in cells/mL and cell viability in percentage are shown on the vertical axis and time in days is shown on the horizontal axis.

A VCD curve 701 and a cell viability curve 703 are calculated from the measurements and cell-specific constants "K" corresponding to the cells. The constants "K" are specific to the candidate cells depicted and differ for other (different) sets of candidate cells.

The curves 701 and 703 may be generated from selected process outputs and cell-specific constants according to the following equations:

$$X_{total} = X_{VCD} + X_{dead} \qquad\qquad (1)$$

$$\frac{dX_{VCD}}{dt} = u_{max} * \frac{1}{\frac{X_{total}}{K_{inhibit}}+1} X_{VCD} \qquad\qquad (2)$$

$$\frac{dX_{dead}}{dt} = K_{dead} \log(X_{VCD}) + K_{toxic} X_{dead} X_{VCD} \qquad\qquad (3)$$

The equations above are an example of how mechanistic modelling could be carried out. Other equations could be used.

With regard to equations (1) to (3) above, $X_{total}$ (total number of cells, alive and dead), $X_{VCD}$ (viable cell density), and $X_{dead}$ (number of dead cells) reflect values of process parameters measured or controlled during a process. $u_{max}$, $K_{dead}$, $K_{toxic}$, and $K_{inhibit}$ are cell-specific constants; equations (1) to (3) may be solved to derive the cell-specific constants according to a conventional optimization method.

Accordingly, as depicted in Fig. 7, $u_{max}$ (maximum growth rate of the cells) is 0.714950953243, $K_{toxic}$ (increase in death rate due to environmental toxicity brought about by dead cells) is 0.00439812036682, $K_{dead}$ (cell death rate) is 0.178336658011, and $K_{inhibit}$ (coefficient characterizing a reduction in growth rate due to the total number of cells) is 108.398985599.

$K_{inhibit}$ reflects the principle that cells grow more slowly when the total number of cells is greater. Thus, $K_{inhibit}$ may grow with cell density (i.e., cells may be inhibited from growing as cell density increases). Equations (1) to (3) reflect the effects of cell density on cell growth, but other effects may also be considered.

Mechanistic modeling may be carried out during correlation of received data in order to fill in missing measurements, e.g., by a plotting one or more curves (as shown in Figure 7) based on existing measurements and known physical characteristics of the cells. Accordingly, the mechanistic modeling may affect values of the selected process parameters and selected process outputs.

The use of cell-specific constants in the context of correlation (e.g., filling in missing data, data smoothing or interpolation) of process parameter values and process outputs via mechanistic modeling (as described above) has the effect of reflecting physical properties of cells when carrying out the correlation. This may lead to more accurate calculation of the scores for the sets of candidate cells (particularly in comparison to approaches that rely exclusively on multivariate statistical approaches such as PCA or PLS), and accordingly, selection of the optimal target cells from the sets of candidate cells.

A process control device 10 (also referred to as a bioreactor system) including an array of vessels 100 (e.g., microscale bioreactors) is shown in Fig. 8. The process control device 10 may be mounted to the deck of a base station in a larger scale process control device. In particular, the process control device 10 may be a microscale process control device suitable for mounting to a macroscale process control device. The macroscale process control device may include vessels having a size that differs from a size of the vessels of the microscale process control device by at least one order of magnitude.

The process control device 10 comprises a base 12, to which is mounted a base plate 13 defining a receiving station 14 for removably receiving a plurality of vessels 100. A clamp plate (not shown) may be removably connected to the base plate 13, in a position overlaying the receiving station 14, via a pair of posts 22 projecting from the upper surface of the base plate 13. The clamp plate may facilitate a drive connection between the drive mechanism of a stirrer 116 (described below).

In the depicted example, the receiving station 14 can hold up to twelve vessels 100 in two rows of six at respective locations 16. In Fig. 8, six vessels 100 are shown in position in their respective vessel receiving locations 16, while six of the vessel receiving locations 16 are shown empty to better illustrate fluid ports 314 a-c in the base plate 13.

The receiving station 14 could be designed to accommodate a greater or lesser number of vessels 100 and the vessels 100 could be arranged in any suitable configuration.

One or more heaters or chillers (not shown) may be located adjacent to the vessel receiving locations 16 to control the temperature of the vessels 100.

With reference to Fig. 8, one of the vessels 100 comprises a chamber 105 for receiving a fluid 107 (e.g., a cell culture solution) having a headspace 109 above. The vessel 100 includes a pipette access port 106, to which a cap 108 is removably attached. The cap 108 is removed for fluids to be pipetted into or out of the vessel 100. A fluid input port 112 may include a filter 114.

The stirrer 116, comprising blades 118, may be rotatably mounted at the base of a vertical shaft 120 within the vessel 100. The upper end of the vertical shaft 120 includes a drive input 124 (e.g., for the drive mechanism, not shown).

A pH sensor spot 126 and a dissolved oxygen (DO) sensor spot 128 are disposed at the bottom of the vessel 100, such that they are able to detect the pH and DO levels of the fluid 107 and to be interrogated from the exterior of the vessel 100.

Venting of the vessel chamber 105 is achieved via a labyrinthine path connecting the chamber 105 to the atmosphere via the stirrer shaft drive input 124. Alternatively, a separate vent port may be provided towards the top of the vessel 100.

A lip 130 may project out to the side of the vessel 100. The lip 130 includes a through port 132b (two optional additional ports 132a and 132c are not shown). A gallery plate 134 is secured above a portion of the top of the vessel 100. The gallery plate 134 includes at least one groove 136b extending to the fluid input port 112 at the top of at least one tube 110b. The gallery plate further includes at least one through port 132b. The lip 130 and the gallery plate 134 together form a rigid ledge projecting to the side of the vessel.

The clamp plate (not shown) may also reinforce a seal between the through port 132b and the fluid ports 314a-c.

A valve assembly 300 is mounted to the underside of the base 12. The valve assembly is received in a cavity of the base station when the process control device 10 is connected to the base station.

In order to carry out a process, the process control device 10 is loaded with vessels 100, each vessel being placed in a respective vessel receiving location within the receiving station 14. When the vessels 100 are inserted into the receiving station 14, the port 132b in the bottom surface of the lip 130 is aligned with and forms a sealed connection with the corresponding receiving station fluid port 314b on the upper surface of the base plate 13.

The respective ports are automatically aligned with one another on insertion by virtue of the defined locations of the vessel receiving station, including the fluid ports 314a-c adjacent thereto, and the rigid ledge, which places the corresponding vessel connection ports 132a-c in alignment with the receiving station fluid ports 314a-c.

## Claims

1. A computer-implemented method for selecting at least one set of target cells from multiple sets of candidate cells, the method comprising:

   receiving (S101, S601) data collected from a plurality of processes, wherein each of the processes produces a distinct set of candidate cells,

       the received data including values of process parameters and process outputs of the processes, each of the process outputs being a product quality attribute or a key performance indicator for selecting the target cells;

   correlating the received data;
   receiving (S603) a selection of the process parameters and a selection of the process outputs;
   receiving (S103, S605) multivariate evaluation criteria for the selected process parameters and/or the selected process outputs, the multivariate evaluation criteria including one or more of the following:

       weights for prioritization;
       prioritization ranges and/or targets, wherein each target is an extremum and/or a target value;

   calculating (S105, S107, S109), via a multivariate selection function, scores for each one of the sets of candidate cells from the correlated data according to the multivariate evaluation criteria;
   ranking (S111) the sets of candidate cells according to the scores; and
   selecting at least one of the sets of candidate cells as the target cells using the ranking.

2. The method of claim 1, wherein the target cells are at least one of the following: a cell line, a cell strain, a clone.

3. The method of claim 1 or 2, wherein the values of the process parameters include time series values, wherein the process parameters were controlled and/or measured during each of the processes.

4. The method of any one of the preceding claims, wherein the received data includes substantially all data from each of the processes.

5. The method of any one of the preceding claims, further comprising:

identifying whether the received data for any of the processes is incomplete, wherein one of the processes is identified as having incomplete data when data is not collected during a portion of the process;
when any of the processes has incomplete data, predicting values for the incomplete data using at least one multivariate technique,
wherein the multivariate technique may include partial least squares regression or interpolation.

6. The method of any one of the preceding claims, wherein the correlating includes verifying and correcting values of the data,

    wherein the correcting comprises revising or excluding values that violate one or more known metabolic dependencies.

7. The method of any one of the preceding claims, further comprising:

    applying mechanistic modelling to the received data to obtain additional values of the process parameters and/or additional process outputs;
    supplementing the received data with the additional values of the process parameters and/or the additional process outputs.

8. The method of any one of the preceding claims, further comprising:

    excluding, from the correlated data, data received from ones of the processes according to exclusion criteria;
    if at least one of the selected process outputs has a corresponding acceptability range, then the exclusion criteria include the corresponding acceptability range for the at least one of the selected process outputs.

9. The method of any one of the preceding claims, wherein the evaluation criteria further comprise:

    - a time based profile of one or more of the process parameters,
    - a profile describing one or more of the process outputs,
    - a trajectory describing time based development of one or more of the process parameters.

10. The method of claim 9, further comprising:

    displaying the correlated data for the selected process parameters and/or the selected process outputs, comprising, displaying correlation patterns for the glycan profiles of the sets of candidate cells.

11. The method of any one of the preceding claims, wherein the selection function includes an objective function, particularly a cost function.

12. The method of any one of the preceding claims, wherein the selection function includes at least one magnifying function, wherein the magnifying function magnifies a distance between values,
wherein each of the prioritization ranges and/or targets has an associated magnifying function,
wherein the magnifying function is non-linear, particularly exponential.

13. The method of any one of the preceding claims, wherein there are at least 5 sets of candidate cells, at least 10 sets of candidate cells, at least 20 sets of candidate cells, at least 30 sets of candidate cells, or at least 50 sets of candidate cells.

14. A computer program comprising computer-readable instructions, which, when loaded and executed on a computer system, cause the computer system to perform operations according to the method of any one of the preceding claims.

15. A process control device (10) for selecting at least one set of target cells from multiple sets of candidate cells, the device (10) comprising:

    a plurality of vessels (100), each of the vessels (100) being configured to contain fluid (107) including one of the sets of candidate cells;
    a robot capable of addressing each of the vessels (100), dispensing fluid to each of the vessels (100), and extracting samples of fluid (107) from each of the vessels (100);

a controller operable to:

control, at least partly in parallel, conditions in each of the vessels;
receive data collected from a plurality of processes, wherein each of the processes produces a distinct set of candidate cells,

the received data including values of process parameters and process outputs of the processes, each of the process outputs being a product quality attribute or a key performance indicator for selecting the target cells;

correlate the received data;
receive a selection of the process parameters and a selection of the process outputs;
receive multivariate evaluation criteria for the selected process parameters and/or the selected process outputs, the multivariate evaluation criteria including one or more of the following:

weights for prioritization;
prioritization ranges and/or targets, wherein each target is an extremum and/or a target value;

calculate, via a multivariate selection function, scores for each one of the sets of candidate cells from the correlated data according to the multivariate evaluation criteria;
rank the sets of candidate cells according to the scores; and
select at least one of the sets of candidate cells as the target cells using the ranking.

16. The device (10) of claim 15, wherein the each of the vessels (100) has at least one of the following characteristics:

it is a bioreactor or a microbioreactor;
it includes stirring means (116) for stirring its contents, wherein the stirring means may be an impeller;
it includes delivery means (110b) for gas delivery, wherein the delivery means may include a sparge tube;
it includes sensing means (126, 128) for measuring at least one of the following: pH, dissolved oxygen, temperature;
it has a volume of: at least 1 ml, at least 10 ml, at least 15 ml, less than 2000 L, less than 1000 L, less than 100 L, less than 50 L, less than 5 L, less than 1 L;
it is disposable.

# FIG 1

| Receive process parameter values and process outputs | S101 |

↓

| Receive evaluation criteria, including at least one target | S103 |

↓

| Determine distance between a value/output and the target | S105 |

↓

| Magnify the distance via a continuous, non-linear function | S107 |

↓

| Modify magnified distance based on priority | S109 |

↓

| Aggregate modified distances corresponding to each paramater value and process output | S111 |

# FIG 2

EP 3 640 946 A1

FIG 3

EP 3 640 946 A1

# FIG 4

Clone selection with quality.M3 (PCA-X)

# FIG 5

Quality profile clone screening data.M1 (PCA-X)

## FIG 6

| S601 | S603 | S605 | S607 | S609 |
|------|------|------|------|------|
| IMPORT | SET CRITERIA | CLONE SELECTION | ANALYSIS | REPORT |

| Process data | Variable list | Variable filters | Color by | Audit trall |
|---|---|---|---|---|
| Quality data | • Min, max, last, integral, average value | • Minmize, maximize, target, exclude | Size by | • Created project |
| • One value per clone • Spectral | | • Min, target and max values | Compare selections | • Revision profile • Revision clones • Change list |
| Missing data handling | • Include exclude variables and values | • Weight / priority | Calculate predicted values | |
| Visual quality control | Data table | Ranking list | MVDA-Scores | Recommendations |
| Exlude data | Clone list | • Desirability function • Sort by ◦ Ranking ◦ Group ◦ Qualified ◦ Not qualified ◦ Top X clones | Correlation analysis | Explanations |
| Group data | • Include & exclude clones | | | |
| Match data | Pre-filter clones | | | |
| Create project | • Acceptable range | Criteria profile | | |
| | Raw data visualisation | • Save profile • Share profile | | |

EP 3 640 946 A1

FIG 7

FIG 8

EP 3 640 946 A1

FIG 9

Europäisches
Patentamt

European
Patent Office

Office européen
des brevets

# EUROPEAN SEARCH REPORT

Application Number

EP 18 20 0386

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 270 129 A2 (AUTOMATION PARTNERSHIP CAMBRIDGE LTD [GB]) 5 January 2011 (2011-01-05) * abstract * * paragraphs [0055], [0056] * * paragraph [0061] - paragraph [0066] * * paragraph [0095] - paragraph [0101] * * paragraphs [0105], [0106] * * claims 1,20,21 * * figures 1,3b * | 1-16 | INV. G16B40/00 ADD. G16C20/70 |
| X | US 6 673 532 B2 (UNIV MARYLAND [US]) 6 January 2004 (2004-01-06) * figures 1,2,6,7,8 * * column 1, line 60 - column 2, line 7 * * column 2, line 42 - column 3, line 26 * * column 7, line 40 - column 8, line 39 * * claim 1 * | 1-16 | |
| X | EP 2 447 717 A1 (LONZA BIOLOGICS PLC [GB]) 2 May 2012 (2012-05-02) * the whole document * | 1-16 | |
| X | US 2017/355947 A9 (BIOGEN MA INC [US]) 14 December 2017 (2017-12-14) * the whole document * | 1-16 | TECHNICAL FIELDS SEARCHED (IPC) G16B G16C G01N C12N C12Q G05B |
| A | US 2010/191361 A1 (MCCREADY CHRISTOPHER PETER [CA] ET AL) 29 July 2010 (2010-07-29) * the whole document * | 1-16 | |
| A | WO 2017/174580 A1 (BOEHRINGER INGELHEIM RCV GMBH [AT]; SANDOZ AG [AT]) 12 October 2017 (2017-10-12) * the whole document * | 1-16 | |

-/--

The present search report has been drawn up for all claims

| Place of search | Date of completion of the search | Examiner |
|---|---|---|
| The Hague | 24 May 2019 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

<table>
<tr><td>Europäisches Patentamt<br>European Patent Office<br>Office européen des brevets</td><td>**EUROPEAN SEARCH REPORT**</td><td>Application Number<br>**EP 18 20 0386**</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2007/018868 A1 (BIOGEN IDEC INC [US]; MISHRA SOUMITRA [US]) 15 February 2007 (2007-02-15) * the whole document * | 1-16 | |
| A | US 2014/136146 A1 (MCCREADY CHRIS PETER [CA]) 15 May 2014 (2014-05-15) * the whole document * | 1-16 | |
| A | US 2013/268238 A1 (MCCREADY CHRISTOPHER PETER [CA]) 10 October 2013 (2013-10-10) * paragraph [0002] * * claims 1,10-13 * * figure 2 * | 1-16 | |
| A | LI B. ET AL: "Performance monitoring of a mammalian cell based bioprocess using Raman spectroscopy", ANALYTICA CHIMICA ACTA, ELSEVIER, AMSTERDAM, NL, vol. 796, 6 August 2013 (2013-08-06), pages 84-91, XP028711483, ISSN: 0003-2670, DOI: 10.1016/J.ACA.2013.07.058 * the whole document * | 1-16 | |
| A | US 2008/133044 A1 (GRIEB HERBERT [DE] ET AL) 5 June 2008 (2008-06-05) * paragraph [0004] - paragraph [0008] * | 1-16 | |

TECHNICAL FIELDS SEARCHED (IPC)

The present search report has been drawn up for all claims

| Place of search | Date of completion of the search | Examiner |
|---|---|---|
| The Hague | 24 May 2019 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 3 640 946 A1

### ANNEX TO THE EUROPEAN SEARCH REPORT
### ON EUROPEAN PATENT APPLICATION NO.

EP 18 20 0386

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2019

| Patent document cited in search report | | Publication date | Patent family member(s) | | Publication date |
|---|---|---|---|---|---|
| EP 2270129 | A2 | 05-01-2011 | EP | 2270129 A2 | 05-01-2011 |
| | | | EP | 3287517 A1 | 28-02-2018 |
| | | | US | 2011003323 A1 | 06-01-2011 |
| US 6673532 | B2 | 06-01-2004 | AU | 8290801 A | 25-02-2002 |
| | | | CA | 2419474 A1 | 21-02-2002 |
| | | | DE | 01961662 T1 | 03-05-2007 |
| | | | EP | 1309719 A1 | 14-05-2003 |
| | | | EP | 2371942 A2 | 05-10-2011 |
| | | | US | 2002025547 A1 | 28-02-2002 |
| | | | US | 2004121453 A1 | 24-06-2004 |
| | | | WO | 0214539 A1 | 21-02-2002 |
| EP 2447717 | A1 | 02-05-2012 | AU | 2011322921 A1 | 18-04-2013 |
| | | | CA | 2814697 A1 | 03-05-2012 |
| | | | CN | 103189744 A | 03-07-2013 |
| | | | EP | 2447717 A1 | 02-05-2012 |
| | | | EP | 2633313 A1 | 04-09-2013 |
| | | | ES | 2434737 T3 | 17-12-2013 |
| | | | ES | 2535608 T3 | 13-05-2015 |
| | | | HK | 1181849 A1 | 02-09-2016 |
| | | | JP | 5746768 B2 | 08-07-2015 |
| | | | JP | 2013545449 A | 26-12-2013 |
| | | | KR | 20140026335 A | 05-03-2014 |
| | | | SG | 189087 A1 | 31-05-2013 |
| | | | TW | 201231661 A | 01-08-2012 |
| | | | US | 2013295596 A1 | 07-11-2013 |
| | | | US | 2017242029 A1 | 24-08-2017 |
| | | | WO | 2012055554 A1 | 03-05-2012 |
| US 2017355947 | A9 | 14-12-2017 | EP | 3164481 A2 | 10-05-2017 |
| | | | US | 2017130186 A1 | 11-05-2017 |
| | | | WO | 2016004322 A2 | 07-01-2016 |
| US 2010191361 | A1 | 29-07-2010 | CN | 102301289 A | 28-12-2011 |
| | | | DE | 112010000703 T5 | 20-06-2013 |
| | | | GB | 2479850 A | 26-10-2011 |
| | | | JP | 2012515984 A | 12-07-2012 |
| | | | KR | 20110110341 A | 06-10-2011 |
| | | | SG | 173107 A1 | 29-08-2011 |
| | | | TW | 201032009 A | 01-09-2010 |
| | | | US | 2010191361 A1 | 29-07-2010 |
| | | | WO | 2010085479 A1 | 29-07-2010 |
| WO 2017174580 | A1 | 12-10-2017 | AU | 2017248025 A1 | 25-10-2018 |
| | | | CA | 3019979 A1 | 12-10-2017 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 18 20 0386

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2019

| Patent document cited in search report | Publication date | Patent family member(s) | | Publication date |
|---|---|---|---|---|
| | | CN | 109313419 A | 05-02-2019 |
| | | EP | 3440518 A1 | 13-02-2019 |
| | | KR | 20180134942 A | 19-12-2018 |
| | | SG | 11201808401Q A | 30-10-2018 |
| | | WO | 2017174580 A1 | 12-10-2017 |
| WO 2007018868 A1 | 15-02-2007 | US | 2009312851 A1 | 17-12-2009 |
| | | WO | 2007018868 A1 | 15-02-2007 |
| US 2014136146 A1 | 15-05-2014 | NONE | | |
| US 2013268238 A1 | 10-10-2013 | TW | 201351079 A | 16-12-2013 |
| | | US | 2013268238 A1 | 10-10-2013 |
| | | WO | 2013152345 A1 | 10-10-2013 |
| US 2008133044 A1 | 05-06-2008 | AT | 382156 T | 15-01-2008 |
| | | DE | 102004040774 B3 | 27-04-2006 |
| | | EP | 1782137 A1 | 09-05-2007 |
| | | US | 2008133044 A1 | 05-06-2008 |
| | | WO | 2006021427 A1 | 02-03-2006 |

EPO FORM P0459

For more details about this annex : see Official Journal of the European Patent Office, No. 12/82

page 2 of 2